# EUROPEAN PATENT SPECIFICATION

(11) **EP 4 426 243 B1**
(45) Date of publication and mention of the grant of the patent: **27.08.2025**
(21) Application number: 22803049.0
(22) Date of filing: 04.11.2022
(51) Int. Cl.: A61F 5/44

(54) **A CONNECTOR FOR CONNECTING TO AN OSTOMY APPLIANCE**
VERBINDER ZUR VERBINDUNG MIT EINER STOMAVORRICHTUNG
CONNECTEUR DESTINÉ À ÊTRE RELIÉ À UN APPAREIL DE STOMIE

(30) Priority: 05.11.2021 GB 202115928
(43) Date of publication of application: 11.09.2024
(73) Proprietor: Salts Healthcare Limited, West Midlands B7 4AA (GB)
(72) Inventor: SMITH, Lee, Birmingham West Midlands B7 4AA (GB); JASICKA, Ewa, Birmingham West Midlands B7 4AA (GB); WILLIAMS, Kieran, Birmingham West Midlands B7 4AA (GB)
(74) Representative: Forresters IP LLP
(86) International application number: PCT/GB2022/052790
(87) International publication number: WO 2023/079299

(56) References cited:
- WO-A1-2017/127074
- GB-A- 2 207 902
- US-A1- 2003 018 322
- US-A1- 2004 176 731

## Description

### Introduction

Embodiments of the present invention relate to a connector for connecting to an ostomy appliance. Ostomy appliances are well known medicals devices for users that have a stoma. Generally, the appliance has an opening for the stoma to be received in and an adhesive member that contacts the body and holds the appliance in position. Waste exiting the stoma is collected in a collecting volume, which is formed from two flexible walls connected together. Often the appliance includes an outlet valve, so that a user can empty waste that is collected in the collecting volume without having to remove and dispose of the entire appliance.

At times, a user may want to extend the time between emptying their appliance, for example, when they are sleeping. Typically, a larger capacity bag is used to contain waste while the user is sleeping. In this scenario, the outlet valve is connected to the larger capacity bag by a tube, so that the bag fills with waste.

US2004/176731 discloses a urine management system including components in fluid wicking communication with one another - a collection device, a conveyance tube, and a storage container. WO2017/127074 discloses a stabilised catheter tube for insertion into a body of a patient. GB2207902 discloses a tube or bag with a wall having scattered inwardly-projecting members. US2003/018322 discloses a catheter assembly allowing for non-contaminated insertion of a catheter into a urinary canal.

Embodiments of the present invention seek to alleviate one or more problems associated with the prior art.

Further preferred features relating to the aspects of the invention are provided in the appended claims.

### Detailed description

In order that the present disclosure may be more readily understood, preferable embodiments thereof will now be described, by way of example only, with reference to the accompanying drawings, in which:
FIGURE 1 is an illustrative view of an ostomy collection system.
FIGURE 2 is a perspective view of a connector in accordance with an embodiment of the present disclosure;
FIGURE 3 is a partial cut-away view of the connector
FIGURE 4A and 4B are illustrative cross-sectional views of a connector in use;
FIGURE 5 is a cross-sectional view of a connector in accordance with an embodiment of the present disclosure;
FIGURE 6A, 6B, 6C and 6D are cross-sectional views of a connector in accordance with an embodiment of the present disclosure;
FIGURE 7 is a cross-sectional view of a connector in accordance with an embodiment of the present disclosure; and
FIGURE 8 is a side cross-sectional view of a connector in accordance with an embodiment of the present disclosure.

An ostomy device 1 (or ostomy appliance) is configured to attach around a stoma of a user (in the abdominal region), so that waste can be collected as it exits the stoma. The ostomy device includes an adhesive wafer, a collecting volume and an outlet 1a. The adhesive wafer includes an opening through which the stoma passes and an adhesive which is suitable for adhering the wafer to the skin of the user. The collecting volume is formed of two outer walls attached about their periphery to define a volume within. The stoma opening connects to the collecting volume, so that when the device is attached to the user, waste exiting the stoma in the stoma opening is collected in the collecting volume.

The collecting volume is connected to the outlet 1a, which is selectively openable / closeable to drain waste from the collecting volume (into a toilet or other waste receptacle, for example). The outlet 1a may be formed in a "tail" portion of the device which is narrowed compared to the main collecting volume. There are different outlet designs that are designed to accommodate different types / consistencies of waste exiting the ostomy device 1.

Embodiments of the present invention are particularly suited for use with a waste with a high proportion of liquid (e.g. urostomy or ileostomy). In this situation, a valve or tap device is used as an outlet, which allows the waste to be emptied from the ostomy device as desired.

The outlet 1a includes a mechanism that allows further parts of a collection system to be attached. The collection system includes a secondary collection volume 2 that is connected to the ostomy device via a connector 10, so that waste may flow into the ostomy device 1 and through the connector 10 to the secondary collection volume 2.

The connector 10 includes a conduit 12 and a structural member 14. The conduit 12 has an entrance and an exit connected by an internal passage 16 (also called passage 16), through which waste is permitted to flow.

The conduit 12 is formed by a wall having an internal surface 12a and external surface 12b. The internal surface 12a defines the internal passage 16 (in other words, defines a perimeter of the passage 16). The external surface 12b forms the outside of the conduit 12. In some embodiments, the conduit 12 has a substantially constant diameter along its length (i.e. the diameter across the passage 16 is substantially constant). In some embodiments, the wall of the conduit 12 is a constant thickness, so the overall / external diameter of the conduit 12 is also substantially constant.

The internal diameter of the conduit 12 (i.e. the size of the passage) depends on the type of ostomy the connector 10 is designed to be used with. For example, both urostomy and ileostomy typically output a high proportion of liquid waste and, thus, a connector 10 as described here could be appropriate for transporting the waste. However, a urostomy may output waste at a slower rate whereas an ileostomy may output at a much faster rate. Thus, the connector 10 may be altered in size to provide adequate sized passage 16 for differing output rates.

In some embodiments, the internal diameter of the conduit 12 is between around 6mm and 16mm. The internal diameter of the conduit 12 for use in urostomy may be at the low end of the range (i.e. between around 7 and 10mm and preferably around 8mm). Further, the diameter of the conduit 12 for use in ileostomy may be at the high end of the range (i.e. between around 13 to 16mm and preferably around 10mm)
In some examples (such as those illustrated in the figures), the conduit 10 is a flexible tube. The conduit 10 may be made of any suitable material that provides the necessary attributes, for example, a thermoplastic elastomer (TPE) may provide the necessary flexibility.

In some embodiments, the conduit 12 forms a generally cylindrical shape. The flexibility of the conduit 12 allows it to change shape depending on the forces being put on the conduit 12 - however, it should be appreciated that the conduit 12 will return to its generally cylindrical shape once it is no longer being manipulated. For example, the conduit 12 can be bent, crushed and / or twisted, etc. and return to its initial shape once the forces causing that change in shape are removed (and preferably without damage).

A structural member 14 extends along the passage 16. The structural member 16 is present to prevent (or at least inhibit of) the passage 16 from being occluded. This may occur when the connector 10 is under pressure / being compressed, being twisted / bent or otherwise manipulated in a way that causes the passage 16 to become blocked and / or causes waste to be inhibited from flowing through the passage 16.

In some embodiments, the structural member 14 extends along around half of a length of the passage 16. The structural member 14 may be deliberately inserted into the area / region of the passage 16 that is likely to be more susceptible to blockages. For example, the conduit 12 that is located on a generally horizontal surface (i.e. a mattress of a bed) may be more prone to blockage due to the lack of gravitational pull. The structural member 14 may be strategically positioned within the passage 16 to address this potential problem. Alternatively, the structural member 14 may extend along a majority of the length of the passage 16.

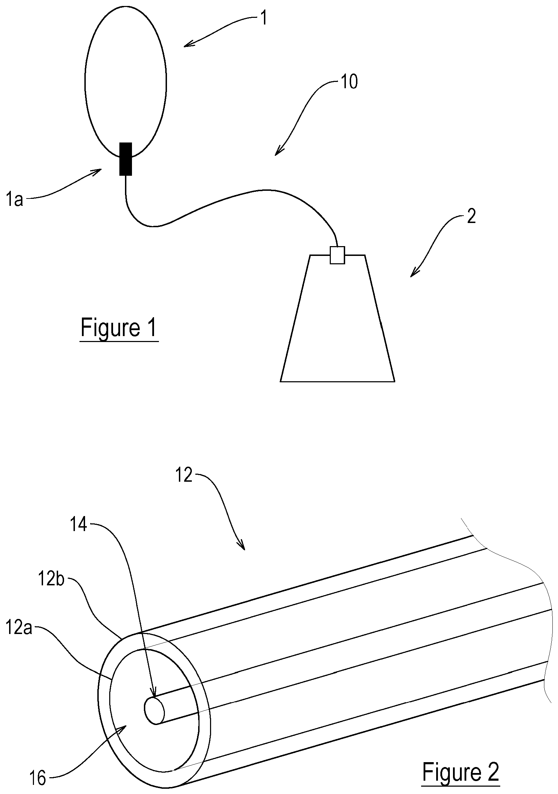

In some embodiments (for example, those illustrated in figures 2 to 4B), the structural member 14 is a filament. In other words, the structural member 14 is a threadlike member or strand that extends along the passage 16. In the present example, the structural member 14 is formed substantially solidly, so that it can resist being compressed (i.e. the structural member 14 does not provide any additional channel or flow path within the passage 16). In other words, the filament is less deformable than the conduit 12.

In some embodiments, the filament is less flexible than the conduit 12. This may also be beneficial because movement of the filament within the conduit 12 (as a result of the difference in flexibility), in use, may act to break up or disturb regions of more viscous waste (i.e. that otherwise may get stuck in the conduit 12 and slow down the passage of waste through the connector 10).

The filament structural member 14 is thin relative to the size of the passage (and preferably is a constant diameter along its entire length), so that it fits within the passage 16 easily. In some embodiments, the diameter of the filament may be between 1 to 2mm and preferably around 1.75mm to 2mm.

Thus, in a urostomy connector 10 as discussed above (e.g. a preferred diameters of around 8mm), the ratio of the diameter of the filament to the diameter of the passage 16 is around 2:8 to 7:32. In other words, the filament is under around 25% of the diameter of the passage 16. In embodiments, the diameter of the filament structural member 14 is not altered for use in an ileostomy connector 10 (e.g. having a diameter of around 10mm), so the ratio of the diameter of the filament to the diameter of the passage 16 may be about 2:10 to 7:40. In other words the filament is under around 20% of the diameter of the passage 16 - the filament may be smaller relative to the passage 16 in this case because the waste being handled may be at a higher rate or have a higher proportion of mucus / more solid waste.

In embodiments, the structural member 14 is made of a flexible material, so that it may flex / bend within the passage 16. For example, the filament may be made from the same material as the conduit 12 (e.g. a TPE) and more specifically from Styrene-ethylene-butylene-styrene (SEBS). However, a more advantageous material for the filament may be polypropylene as it is less flexible than the conduit 12 around it and, as such, acts to prevent the conduit 12 from kinking. Alternative flexible materials are available and preferably the material is PVC free and / or does not have a high coefficient of friction (e.g. is Silicon free). A low coefficient of friction is advantageous because it reduces the adherence between the structural member 14 and waste travelling through the passage 16.

Further, the filament is not absorbent, so waste is able to flow freely through the conduit 12 (around / past the filament) to the secondary collecting volume 2.

In some embodiments, the structural member 14 is not connected to the conduit 12 (or more specifically, is not attached / anchored to the internal surface 12a of the conduit) at any location. In other words, the structural member 14 is not held in a single axial position with respect to the conduit 12.

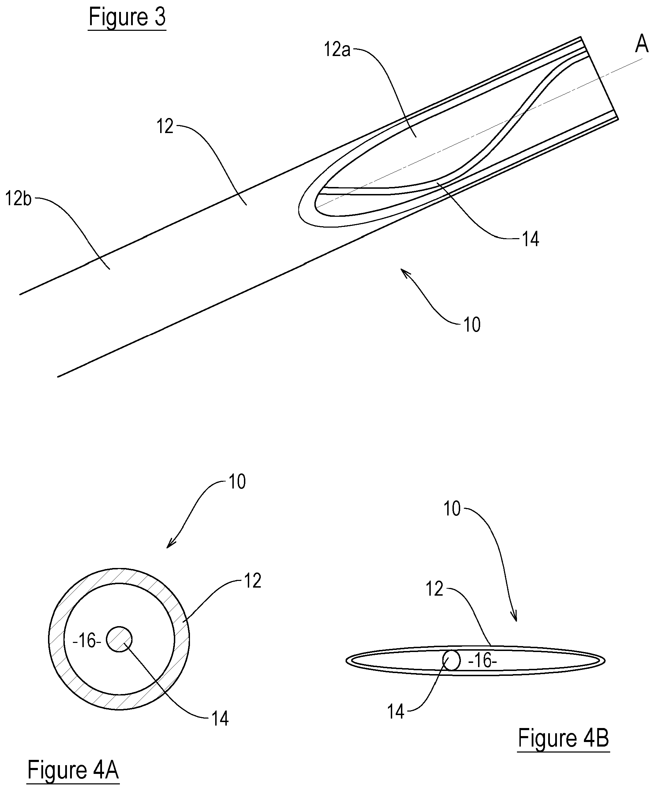

In some embodiments, the structural member 14 can move freely from side to side within the passage 16 (i.e. the structural member 14 can move generally transversely to the direction of the flow of waste through the passage 16). In other words, the conduit 12 has a longitudinal axis (e.g. axis A illustrated on figure 3) along the length of the connector 10 and the structural member 14 can move generally transversely to the axis. This is illustrated in figure 3, for example, where the structural member 14 is shown to form a general sine wave shape that contacts the internal surface 12a on both "sides" of the passage 16.

As mentioned above, the structural member 14 may be unattached to the conduit 12. However, it should be appreciated that even if the structural member 14 were attached to the conduit 12 at two spaced positions along the length of the conduit, this would still allow the structural member 14 to move from side to side to some extent. The more anchor positions the structural member 14 had, the more restricted the radial movement of the structural member 14 would be. In some embodiments, one or more stake welds may be used to prevent the structural member 14 migrating along the conduit 12.

Thus, the structural member 14 may be free to move with respect to the conduit 12 (and relative to the axis A). This movement may be advantageous to maintaining waste transport along the conduit 12. For example, waste may include portions that have higher viscosity and do not flow easily along the conduit 12. The movement of the structural member 14 (particularly movement of the filament from side to side of the passage 16) can be used in this situation to help break down regions of higher viscosity waste and aid the transport process / ensure the waste continues to flow away from the ostomy device 1. Additionally, the user is able to manipulate the conduit 12 to encourage the structural member 14 within to move from side to side and, thus, break down areas of waste with higher viscosity.

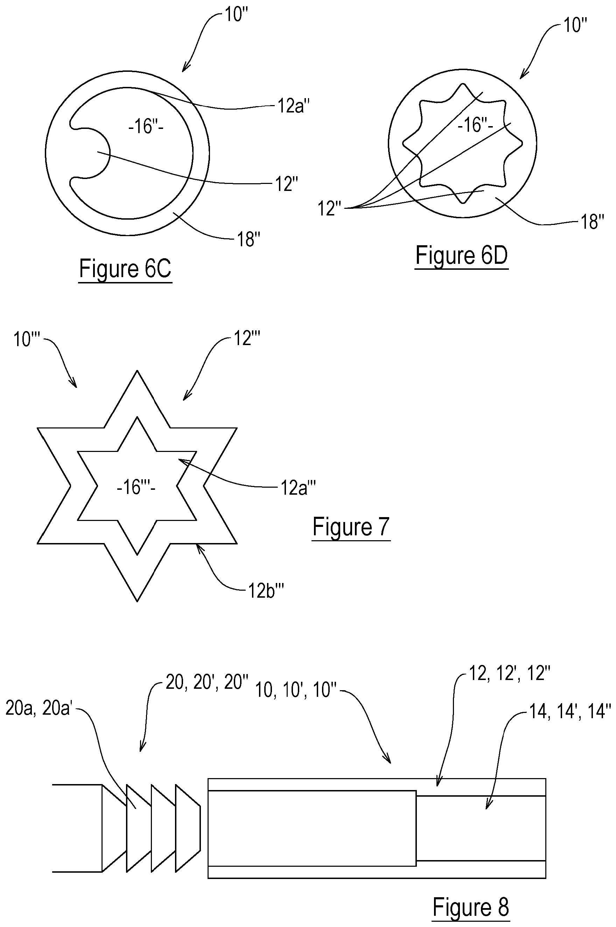

In some embodiments, at least one end of the conduit 12 is adapted to receive an adaptor device 20. The adaptor device 20 is configured to engage (via a liquid tight connection) with one of the outlet 1a of the ostomy device 1 and / or the inlet of the secondary collection volume 2. In the illustrated example in figure 8, the adaptor device 20 is inserted into the entrance and / or exit of the conduit 12. In this example, the adaptor device 20 includes a spigot type fitting 20a that is pushed into the passage 16 and, as such, the adaptor device 20 is held securely to the conduit 12.

This ensures that the connector 10 provides a sealed transport path between the ostomy device 1 and the secondary collection volume 2.

In some embodiments, the adaption to the conduit 12 in order to receive the adaptor device 20 involves removing any parts of the structural member 14 from the "end" of the conduit 12 (i.e. an appropriate distance within the passage 16 adjacent to the entrance / exit). Thus, the conduit 12 has a generally unobstructed bore at one or both ends to allow the adaptor device 20 to be inserted without interruption from any parts present further down the passage 16. In other words, the length of the structural member 14 is shorter than the length of the conduit 12. Thus, the adaptor device 20 extends into the entrance and / or exit without being interrupted by the structural member 14.

It should be appreciated that not all adaptor devices will provide a male connection which is received by the conduit 12. If the adaptor device has a female connection that fits around the conduit 12, then it will not be necessary to adapt the ends of the conduit 12 to accommodate the adaptor device.

In some embodiments, the conduit 12 and the structural member 14 are formed separately (e.g. in two different extrusion lines). Subsequently, the structural member 14 is inserted into the conduit 12 to form the connector 10. In some embodiments, the conduit 12 and the conduit 14 are formed simultaneously in a co-extrusion machine.

An illustrative cross-sectional view of the connector 10 being used is shown in figures 4A and 4B. The figure 4A shows the conduit 12 in its natural round or annular state. The structural member 14 is shown in the centre of the conduit 12 for illustration only - the structural member 14 is able to move from side to side within the passage 16. Waste is free to travel allow the passage 16 around the structural member 14.

Figure 4B shows the conduit 12 when it is being compressed - this could arise because the connector 10 is being bent sharply / kinked and /or because it is being twisted / experiencing a torsional force and / or because it is under a compressing force (e.g. being stepped on). As can be seen in the figure, the conduit 12 no longer has its natural round / annular shape - it is forced into a shape resembling an oval or lanceolate. The structural member 14 within the passage 16 resists deformation and provides a reinforcing function that prevents the conduit 12 from compressing completely (i.e. prevents the internal surface 12a from either "side" of the conduit 12 from coming into contact). Thus, the flow path through the passage 16 is prevented (or at least inhibited) from closing / becoming occluded.

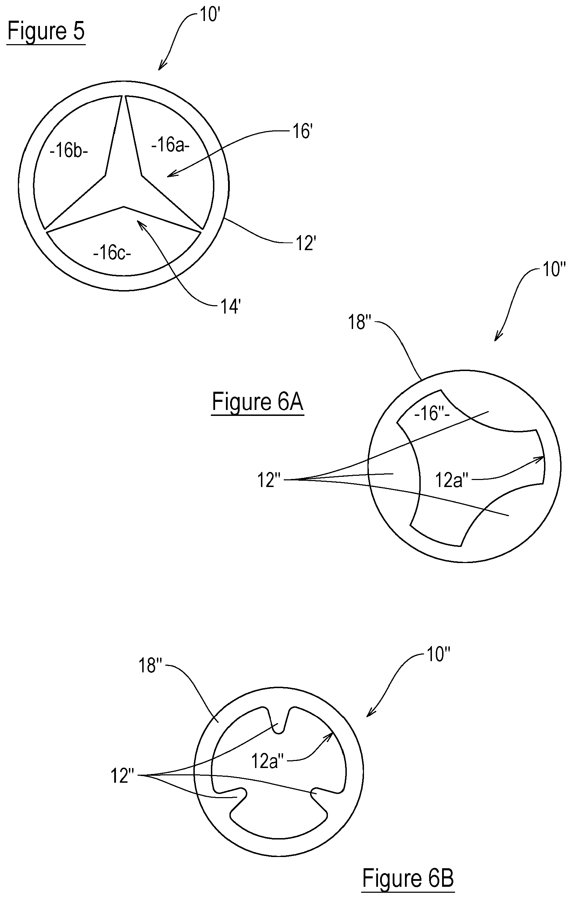

An alternative structural member is illustrated in figure 5. Where features are specifically described here and are analogous to features that have already been discussed, the reference numbers are the same with the addition of a prime symbol (e.g. 10 becomes 10'). The example illustrated in figure 5 only provides a cross-sectional view of the connector 10'. However, it should be appreciated that many of the features already described but not explicitly illustrated in figure 5 are still applicable, such as the materials used and the adaptions provided for an adaptor device 20'.

In some embodiments, the structural member 14' is formed to split the passage 16' into two or more channels. In other words, a specific shape of structural member 14' is chosen, so that it can be positioned in the passage 16'. The passage 16' is split by the structural member 14' to provide multiple lumens.

In some embodiments, the structural member 14' extends radially across the passage 16'. For example, the structural member 14' contacts an interior surface 12a' of the conduit 12' in at least two locations.

In the example illustrated in figure 5, the structural member 14' is formed with a cross-sectional shape of a three-pointed star. In cross section (i.e. across the conduit 12'), the points of the star extend outwards to contact the internal surface 12a' of the conduit 12' wall. In other words, the structural member 14' splits the passage 16' into three channels 16a, 16b, 16c. It will be appreciated that other shapes of structural member 14' could be used and achieve a similar outcome of multiple channels provided within the passage 16'. For example, a star shape having more than three points could be used to split the passage 16' into more than three channels (in other words, a four-pointed or five-pointed star shape is envisaged, which would split the conduit 12' into four or five channels, respectively). Furthermore, a structural member that engages the internal surface of the conduit 12' at only two locations could be used to split the passage 16' in two.

It should be appreciated that since the structural member 14' is flexible the surfaces contacting the internal surface 12a' of the conduit 12' does not provide a seal as such, so each channel formed is not necessarily sealed from the other channels. When the conduit 12' is twisted / bent / compressed / etc. the structural member 14' functions to strengthen the conduit 12' and resists deformation. Further, the portions of the structural member 14' that extend outwards to the internal surface of the conduit 12' (e.g. the points of the star shape) may inhibit major deformation and maintain one or more of the channels 16a, 16b, 16c within the passage 16' open for waste to flow.

An alternative embodiment of the disclosure is illustrated in figures 6A. Where features are specifically described here and are analogous to features that have already been discussed, the reference numbers are the same with the addition of a double prime symbol (e.g. 10 becomes 10"). New features also have a double prime but the references have not been used before. The example illustrated in figure 6 only provides a cross-sectional view of the connector 10". However, it should be appreciated that many of the features already described but not explicitly illustrated in figure 6 are still applicable, such as the materials used and the adaptions provided for an adaptor device.

The conduit 12" defines the internal passage 16" for the waste to flow along. In this example, the internal surface 12a" includes an inwardly projecting member 18".

In some embodiments, there are two or more inwardly projecting members 18" (three in the illustrated example), which are spaced around the internal surface 12a" of the conduit 12". The inwardly projecting members 18" may be equally spaced around the internal surface 12a" of the conduit 12".

In some embodiments, the inwardly projecting member(s) 18" extend longitudinally along the passage 16" (optionally, the inwardly projecting member(s) 18" extend along a majority of the total length of the conduit 12"). In other words, the inwardly projecting member 18" is present from the entrance to the exit of the conduit 12". Although it should be appreciated that they may not be directly adjacent the entrance and / or exit to accommodate an adaptor device 20" as already discussed above.

In the illustrated embodiment, the inwardly projecting members 18" are raised and smooth bumps that extend inwards from the internal surface 12a" of the conduit 12". However, it should be appreciated that this need not be the case and the shape of the projecting members 18" could be generally rectangular or another shape (discussed below). The important aspect is that the internal projecting member(s) 18" prevent or at least inhibit the passage 16" being occluded under compression / other manipulation as discussed in detail elsewhere in the description.

The examples illustrated in figure 6B to 6D are similar to that described above in that they include one or more projecting member(s) 18" extending radially inwards. The example in figure 6B also includes three inwardly projecting members 18". The inwardly projecting members 18" are generally triangular with a point extending furthest into the passage 16".

The example in figure 6C provides only a single inwardly projecting member 18". The member 18" is generally circular in cross-section with a portion attached to the internal surface 12a" of the conduit.

In the example in figure 6D, the internal surface 12a" of the conduit 12" is continuously undulate. This is formed from eight inwardly projecting members 18" but it should be appreciated that this could be adjusted depending on the size of the members 18" required and the size of the conduit 12".

An alternative conduit 12‴ is illustrated in figure 7. Where features are specifically described here and are analogous to features that have already been discussed, the reference numbers are the same with the addition of a triple prime symbol (e.g. 12 becomes 12‴). New features also have a triple prime but the references have not been used before. The example illustrated in figure 7 only provides a cross-sectional view of the connector 12‴. However, it should be appreciated that many of the features already described but not explicitly illustrated in figure 7 are still applicable, such as the materials used.

In this embodiment, the conduit 12'" includes an entrance and an exit connected by an internal passage 16'" through which waste is permitted to flow. The conduit 12'" has a polygonal cross-sectional shape. In some embodiments (as illustrated in figure 7), the conduit 12'" forms a six pointed star shape in cross-section. However, it should be appreciated that a different shape could be used (for example, a star with a different number of points or another polygonal shape).

The conduit 12'" is formed by a wall having an internal surface 12a‴ and external surface 12b‴. The internal surface 12a‴ defines the internal passage 16‴. The external surface 12b‴ forms the outside of the conduit 12‴. In the present example, the wall is of substantially constant thickness along its length, so the diameter of the passage 16'" and conduit 12'" is substantially constant along the majority of the connector 10"'. In other words, both the internal shape of the wall and the external shape of the wall has the same cross-sectional shape.

In some embodiments, the conduit 12'" is formed of a suitable flexible material which permits an adaptor device to be inserted in the entrance and / or the exit. In this example, the conduit 12ʺ' is stretched / deformed to an annular shape to accept the adaptor device (not shown) (for example, a spigot fitting that is pushed into the deformed conduit 12‴). Additionally, the conduit 12'" seals against the adaptor device to inhibit egress of liquid between the conduit 12"' and the adaptor device.

Embodiments of the disclosure described here provide a connector 10, 10', 10", 10"' that resists occlusion in the event the conduit 12, 12', 12", 12ʺ′ is compressed, twisted, bent, kinked, and/or other type of manipulation that could happen during use. Such occlusion could result waste becoming stuck in the passage 16, 16', 16", 16"' for longer than necessary and / or backing up the passage 16, 16', 16", 16"' and resulting in a leak at an earlier connection point (i.e. between the ostomy device and connector or in the ostomy device itself), which can be distressing to a user and / or cause a user to lose confidence in the system / connector provided.

## Claims

1. A connector (10) for connecting to an ostomy appliance (1) **characterised in that** it includes:
a conduit (12), forming a generally cylindrical shape when the conduit is not being manipulated, and having an entrance and an exit connected by an internal passage (16) through which waste is permitted to flow, and wherein
a structural member (14) extends along the passage, and is provided by a threadlike flexible filament.

2. A connector (10) according to claim 1 wherein the structural member (14) extends along around half of a length of the passage (16) and optionally, the structural member (14) extends along a longer length.

3. A connector (10) according to any of the previous claims wherein a longitudinal axis is defined along a length of the conduit (12) and the structural member (14) is permitted to move generally transversely to the axis.

4. A connector (10) according to claim 3 wherein the structural member (14) is free to move within the conduit (12).

5. A connector according to claims 3 or 4 wherein the structural member (14) is unanchored to any part of the conduit (12).

6. A connector (10) according to any one of the preceding claims wherein the filament is between around 1mm and 2mm in diameter and preferably around 1.75mm.

7. A connector (10) according to any one of the preceding claims wherein a ratio of the diameter of the conduit (12) to a diameter of the filament is around 32 to 7 or around 40 to 7.

8. A connector (10) according to any of the preceding claims wherein the conduit (12) has a substantially constant interior diameter along its length.

9. A connector (10) according to any of the preceding claims wherein the conduit (12) is made from a thermoplastic elastomer (TPE) and / or the structural member (14) is made from a TPE and optionally specifically Styrene-ethylene-butylene-styrene (SEBS).

10. A connector (10) according to any of the preceding claims wherein at least one end of the conduit (12) is adapted to receive an adaptor device that is inserted into the entrance and / or exit of the conduit (12).

11. A connector (10) according to claim 10 wherein the length of the structural member (14) is shorter than the length of the conduit (12), so that the adaptor device extends into the entrance and / or exit without being interrupted by the structural member (14).

12. An ostomy collection system including an ostomy appliance (1), a further collecting device and a connection mechanism, the ostomy appliance (1) having:
an adhesive member for attaching around a stoma of a user,
a collecting volume for storing waste, and
an outlet for waste to exit the collecting volume,
the further collecting device including:
an inlet which is connected to a storage receptacle, and
the connection mechanism including a connector (10) according to any of the preceding claims.

## Patentansprüche

1. Verbinder (10) zur Verbindung mit einer Stomavorrichtung (1), **dadurch gekennzeichnet, dass** er einschließt:
eine Leitung (12), die eine allgemein zylindrische Form bildet, wenn die Leitung nicht manipuliert wird, und einen Eingang und einen Ausgang aufweist, die durch einen internen Durchtritt (16) verbunden sind, durch den Abfallstoffe strömen dürfen, und wobei
sich ein Strukturelement (14) entlang des Durchtritts erstreckt und durch ein fadenartiges flexibles Filament bereitgestellt ist.

2. Verbinder (10) nach Anspruch 1, wobei sich das Strukturelement (14) entlang etwa der Hälfte einer Länge des Durchtritts (16) erstreckt und optional sich das Strukturelement (14) entlang einer längeren Länge erstreckt.

3. Verbinder (10) nach einem der vorhergehenden Ansprüche, wobei eine Längsachse entlang einer Länge der Leitung (12) definiert ist und dem Strukturelement (14) ermöglicht ist, sich allgemein quer zu der Achse zu bewegen.

4. Verbinder (10) nach Anspruch 3, wobei sich das Strukturelement (14) frei innerhalb der Leitung (12) bewegen kann.

5. Verbinder nach den Ansprüchen 3 oder 4, wobei das Strukturelement (14) an einem beliebigen Teil der Leitung (12) unverankert ist.

6. Verbinder (10) nach einem der vorhergehenden Ansprüche, wobei das Filament einen Durchmesser zwischen etwa 1 mm und 2 mm und vorzugsweise von etwa 1,75 mm aufweist.

7. Verbinder (10) nach einem der vorhergehenden Ansprüche, wobei ein Verhältnis des Durchmessers der Leitung (12) zu einem Durchmesser des Filaments etwa 32 zu 7 oder etwa 40 zu 7 beträgt.

8. Verbinder (10) nach einem der vorhergehenden Ansprüche, wobei die Leitung (12) einen im Wesentlichen konstanten Innendurchmesser entlang ihrer Länge aufweist.

9. Verbinder (10) nach einem der vorhergehenden Ansprüche, wobei die Leitung (12) aus einem thermoplastischen Elastomer (TPE) hergestellt ist und/oder das Strukturelement (14) aus einem TPE und optional spezifisch Styrol-Ethylen-Butylen-Styrol (SEBS) hergestellt ist.

10. Verbinder (10) nach einem der vorhergehenden Ansprüche, wobei mindestens ein Ende der Leitung (12) dazu eingerichtet ist, ein Adaptergerät aufzunehmen, das in den Eingang und/oder den Ausgang der Leitung (12) eingeführt wird.

11. Verbinder (10) nach Anspruch 10, wobei die Länge des Strukturelements (14) kürzer als die Länge der Leitung (12) ist, so dass sich das Adaptergerät in den Eingang und/oder den Ausgang erstreckt, ohne durch das Strukturelement (14) gestört zu werden.

12. Stomasammelsystem, einschließend eine Stomavorrichtung (1), eine weitere Sammelvorrichtung und einen Verbindungsmechanismus, wobei die Stomavorrichtung (1) aufweist:
ein Klebeelement zum Anbringen um das Stoma eines Benutzers,
ein Sammelvolumen zum Aufbewahren von Abfallstoffen und
einen Auslass, damit Abfallstoffe aus dem Sammelvolumen austreten können,
wobei die weitere Sammelvorrichtung einschließt:
einen Einlass, der mit einem Aufbewahrungsbehälter verbunden ist, und
wobei der Verbindungsmechanismus einen Verbinder (10) nach einem der vorhergehenden Ansprüche

## Revendications

1. L'invention concerne un connecteur (10) prévu pour être relié à un appareillage de stomie (1) **caractérisé en ce qu'**il comprend :
un conduit (12) de forme généralement cylindrique lorsqu'il n'est pas manipulé et comprenant une entrée et une sortie reliées par un passage interne (16) à travers lequel les déchets peuvent s'écouler, et dans lequel
un élément structurel (14) s'étend le long du passage, et comprend un filament filiforme flexible.

2. Connecteur (10) selon la revendication 1 dans lequel l'élément structurel (14) s'étend le long d'environ la moitié d'une longueur du passage (16) et, de façon facultative, l'élément structurel (14) s'étend le long d'une plus grande longueur.

3. Connecteur (10) selon l'une quelconque des revendications précédentes dans lequel un axe longitudinal est défini le long d'une longueur du conduit (12) et l'élément structurel (14) peut se déplacer de façon généralement transversale par rapport à l'axe.

4. Connecteur (10) selon la revendication 3 dans lequel l'élément structurel (14) peut se déplacer librement à l'intérieur du conduit (12).

5. Connecteur selon les revendications 3 ou 4 dans lequel l'élément structurel (14) n'est pas ancré sur une partie quelconque du conduit (12).

6. Connecteur (10) selon l'une quelconque des revendications précédentes dans lequel le diamètre du filament mesure entre environ 1mm et 2mm et de préférence environ 1,75mm.

7. Connecteur (10) selon l'une quelconque des revendications précédentes dans lequel un rapport entre le diamètre du conduit (12) et un diamètre du filament est d'environ 32 à 7 ou d'environ 40 à 7.

8. Connecteur (10) selon l'une quelconque des revendications précédentes dans lequel le diamètre intérieur du conduit (12) est sensiblement constant sur sa longueur.

9. Connecteur (10) selon l'une quelconque des revendications précédentes dans lequel le conduit (12) est fabriqué dans un élastomère thermoplastique (TPE) et/ou l'élément structurel (14) est fabriqué dans un TPE et, de manière facultative, spécifiquement en styrène-éthylène-butylène-styrène (SEBS).

10. Connecteur (10) selon l'une quelconque des revendications précédentes dans lequel au moins une extrémité du conduit (12) est adaptée pour recevoir un dispositif adaptateur à insérer dans l'entrée et/ou la sortie du conduit (12).

11. Connecteur (10) selon la revendication 10 dans lequel la longueur de l'élément structurel (14) est inférieure à celle du conduit (12), de sorte que le dispositif adaptateur s'étend dans l'entrée et/ou la sortie sans être interrompu par l'élément structurel (14).

12. Système de collecte de stomie comprenant un appareillage de stomie (1), un autre dispositif de collecte et un mécanisme de connexion, l'appareillage de stomie (1) ayant :
un élément adhésif destiné à être fixé autour d'une stomie d'un utilisateur,
un volume de collecte pour stocker des déchets, et
une sortie pour les déchets pour évacuer le volume de collecte,
l'autre dispositif de collecte comprenant :
une entrée connectée à un réceptacle de stockage, et le mécanisme de connexion comprenant un connecteur (10) selon l'une quelconque des revendications précédentes
